# EUROPEAN PATENT APPLICATION

(11) **EP 4 583 362 A1**
(43) Date of publication of application: **09.07.2025**
(21) Application number: 24150606.2
(22) Date of filing: 08.01.2024
(51) Int. Cl.: H02J 50/00, H02J 50/10, G01R 33/28, G01R 33/02, A61B 5/055, A61B 5/00, A61B 5/01, A61B 5/021, A61B 5/1455

(54) **MAGNETIC ENVIRONMENT COMPATIBLE WIRELESS CHARGING**

(71) Applicant: Koninklijke Philips N.V., 5656 AG Eindhoven (NL)
(72) Inventor: BRADY, Leigh, Eindhoven (NL)
(74) Representative: Philips Intellectual Property & Standards

(57) **Abstract**

A reconfigurable wireless charger component for use in a magnetic resonance environment includes at least one reconfigurable conductive coil (110, 210, 310, 410) configured to have a first physical configuration for recharging when distal from a magnetic system and to have a second physical configuration when close to the magnetic system. The reconfigurable charger is configured to wirelessly recharge a device (D) in the first physical configuration. The second physical configuration provides a reduction in induced current in the at least one reconfigurable conductive coil compared to the first physical configuration, e.g. by reducing a loop area of the inductive loop in the reconfigurable conductive coil, wherein the reduction of the loop area is enabled by opening the inductive loop or changing topology of the inductive loop.

## Description

### FIELD OF THE INVENTION

The invention relates to a reconfigurable wireless charger component, a system, a method, and computer program, for wireless recharging of battery powered transportable devices used in a magnetic environment.

### BACKGROUND OF THE INVENTION

Patient monitoring in a magnetic resonance (MR) environment typically consists of the patient being tethered to battery powered transportable devices and to a cart type monitoring system. The monitoring system may measure some parameters and the transportable devices may measure other parameters. The monitoring system communicates wirelessly with the transportable devices. The monitoring system may measure parameters such as, for example, temperature, intra-arterial blood pressure (IBP), and anesthetic agents. The transportable devices used during MR imaging may include modules and/or telemetry devices for measuring other parameters such as, for example, oxygen saturation (SpO2), and electrocardiogram (ECG). These transportable devices may be connected to the patient with a cable and a patient accessory, and are oriented at the foot of the patient, at the side of the patient or on the patient during the MR imaging.

The transportable devices need to be recharged since they are battery powered. Recharging is currently performed using a wired connection. Standard wireless recharging methods use conductive coils for inductive coupling and cannot be implemented in MR compatible devices because the large magnetic fields in the MR environment would induce large currents in the conductive coils in a transportable device, causing heating and damage to the transportable device due to over voltage and over current. The currently-used wired connection has the disadvantage that the metal contacts need to be inaccessible to being touched by patients and users. Additionally, the wired connection must be compatible with cleaning requirements. Accordingly, the wired connection presents mechanical design challenges.

As reference for the context of magnetic resonance systems, Gauss levels at typical MR scanners vary based on the Tesla strength of the magnet for the MR scanners. For a 3 Tesla magnet, the Gauss levels are approximately 30,000 gauss at the iso center of the magnet bore, and 15,000 gauss at the face of the magnet bore. For a 1.5 Tesla magnet, the Gauss levels are approximately 15,000 gauss at the iso center of the magnet bore, and 10,000 gauss at the face of the magnet bore. One gauss corresponds to 10⁻⁴ tesla (T), the International System Unit. Wireless recharging of transportable devices would rely on inductive coupling between a conductive coil in a charger and a conductive coil in a transportable device, and this would induce the large currents that result in creating undesirable energy and heating and damage in the transportable device due to the over voltage and over current. For this reason, wireless recharging of transportable devices that are to be used in the MR environment has been discounted.

Accordingly, battery powered transportable devices used in the MR environment need new mechanisms for wireless recharging.

### SUMMARY OF THE INVENTION

The invention is defined by the independent claims. Advantageous embodiments are defined in the dependent claims.

According to an embodiment of the present disclosure, a reconfigurable wireless charger component suitable for being present in a magnetic environment includes at least one reconfigurable conductive coil. The reconfigurable conductive coil(s) is or are configured to have a first physical configuration for recharging when distal from a magnetic system such as a magnetic resonance imaging system, and to have a second physical configuration when close to the magnetic system, such as in the bore of the magnetic resonance imaging system. The reconfigurable charger is configured to wirelessly recharge a peripheral device in the second physical configuration. The second physical configuration provides a reduction in induced current in the at least one reconfigurable conductive coil compared to the first physical configuration.

According to another embodiment of the present disclosure, a system for reconfigurable charging includes such a reconfigurable wireless charger component.

According to another embodiment of the present disclosure, a method of operating a reconfigurable wireless charger component in a magnetic environment includes configuring at least one reconfigurable conductive coil to have a first physical configuration for recharging when distal from a magnetic system. The method also includes configuring the at least one reconfigurable conductive coil to have a second physical configuration when close to the magnetic system. The method further includes wirelessly recharging a peripheral device via the at least one reconfigurable conductive coil in the first physical configuration. The second physical configuration provides a reduction in induced current in the at least one reconfigurable conductive coil compared to the first physical configuration.

According to another embodiment of the present disclosure, a computer program product is provided comprising instructions which, when the program is executed by a system according to the invention, cause the system to carry out the method of the invention. The computer program product may be downloaded from a server, e.g. via the internet. Alternatively, the computer program product may be stored on a computer-readable memory, like a CD, DVD, or a USB stick.

These and other aspects of the invention will be apparent from and elucidated with reference to the embodiments described hereinafter.

### BRIEF DESCRIPTION OF THE DRAWINGS

The example embodiments are best understood from the following detailed description when read with the accompanying drawing Figures. It is emphasized that the various features are not necessarily drawn to scale. In fact, the dimensions may be arbitrarily increased or decreased for clarity of discussion. Wherever applicable and practical, like reference numerals refer to like elements.
FIG. 1A illustrates a reconfigurable charger for a device being powered in a first state, in accordance with a representative embodiment.
FIG. 1B illustrates the reconfigurable charger for a device being powered of FIG. 1A in a second state, in accordance with a representative embodiment.
FIG. 2A illustrates a reconfigurable charger for a device being powered in a first state, in accordance with a representative embodiment.
FIG. 2B illustrates a reconfigurable charger for a device being powered of FIG. 2A in a second state, in accordance with a representative embodiment.
FIG. 3A illustrates a reconfigurable charger for a device being powered in a first state, in accordance with a representative embodiment.
FIG. 3B illustrates a reconfigurable charger for a device being powered of FIG. 3A in a second state, in accordance with a representative embodiment.
FIG. 4A illustrates a reconfigurable charger for a device being powered in a first state, in accordance with a representative embodiment.
FIG. 4B illustrates a reconfigurable charger for a device being powered of FIG. 4A in a second state, in accordance with a representative embodiment.
FIG. 5 illustrates a system for a reconfigurable charger for a device being powered, in accordance with a representative embodiment.
FIG. 6 illustrates a system for a reconfigurable charger for a device being powered, in accordance with a representative embodiment.
FIG. 7 illustrates a method of operation for a reconfigurable charger for a device being powered, in accordance with a representative embodiment.

### DESCRIPTION OF EMBODIMENTS

In the following detailed description, for the purposes of explanation and not limitation, representative embodiments disclosing specific details are set forth in order to provide a thorough understanding of embodiments according to the present teachings. However, other embodiments consistent with the present disclosure that depart from specific details disclosed herein remain within the scope of the appended claims. Descriptions of known systems, devices, materials, methods of operation and methods of manufacture may be omitted so as to avoid obscuring the description of the representative embodiments. Nonetheless, systems, devices, materials and methods that are within the purview of one of ordinary skill in the art are within the scope of the present teachings and may be used in accordance with the representative embodiments. It is to be understood that the terminology used herein is for purposes of describing particular embodiments only and is not intended to be limiting. Definitions and explanations for terms herein are in addition to the technical and scientific meanings of the terms as commonly understood and accepted in the technical field of the present teachings.

It will be understood that, although the terms first, second, third etc. may be used herein to describe various elements or components, these elements or components should not be limited by these terms. These terms are only used to distinguish one element, configuration or component from another element, configuration or component. Thus, a first element, configuration or component discussed below could be termed a second element, configuration or component without departing from the teachings of the inventive concept.

As used in the specification and appended claims, the singular forms of terms 'a', 'an' and 'the' are intended to include both singular and plural forms, unless the context clearly dictates otherwise. Additionally, the terms "comprises", and/or "comprising," and/or similar terms when used in this specification, specify the presence of stated features, elements, and/or components, but do not preclude the presence or addition of one or more other features, elements, components, and/or groups thereof. As used herein, the term "and/or" includes any and all combinations of one or more of the associated listed items.

Unless otherwise noted, when an element or component is said to be "connected to", "coupled to", or "adjacent to" another element or component, it will be understood that the element or component can be directly connected or coupled to the other element or component, or intervening elements or components may be present. That is, these and similar terms encompass cases where one or more intermediate elements or components may be employed to connect two elements or components. However, when an element or component is said to be "directly connected" to another element or component, this encompasses only cases where the two elements or components are connected to each other without any intermediate or intervening elements or components.

The present disclosure, through one or more of its various aspects, embodiments and/or specific features or sub-components, is thus intended to bring out one or more of the advantages as specifically noted below.

As described herein, wireless recharging may be provided for transportable devices used in the MR environment using inductive coupling. Reconfigurable conductive coils can be used to enable the wireless recharging. The wireless recharging described herein may prevent the damage that would result from an inductive loop in a conductive coil in the MR environment by changing the properties of the inductive loop in the conductive coil such that it can operate like a standard inductive loop in the conductive coil when recharging away from the MR environment yet generate zero or near zero current, voltage and heat when configured for operations in the MR environment.

As described in embodiments herein, reducing the loop area of the inductive loop in the reconfigurable conductive coil 110, 210, 310, 410 of a reconfigurable charger e.g., to zero or near zero enables safe use of the battery powered transportable devices in the MR environment. The reduction of the loop area of the inductive loop in the reconfigurable conductive coil 110, 210, 310, 410 is enabled using a mechanism within the battery powered transportable device to open the inductive loop or change topology of the inductive loop. The inductive loop may be restored for charging wirelessly when outside of the MR environment. Reducing the loop area of the inductive loop in the conductive coil renders battery powered transportable devices safe for use in the MR environment, while restoring the loop area of the inductive loop in the reconfigurable conductive coil 110, 210, 310, 410 enables the wireless recharging outside of the MR environment.

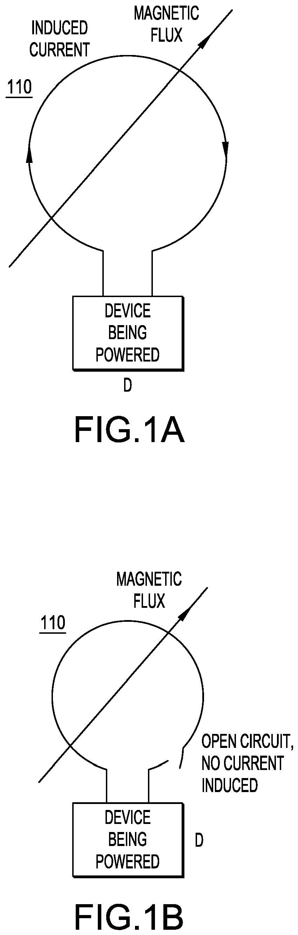

FIG. 1A illustrates a reconfigurable charger for a device being powered in a first state, in accordance with a representative embodiment. More precisely, Fig. 1A, like other figures in this disclosure, shows the wireless charger component at the secondary side of the wireless charger.

FIG. 1A includes a reconfigurable conductive coil 110 attached to a device being powered D. The reconfigurable conductive coil 110 may be considered an element of the device being powered D, or may be considered to be separate and separable from the device being powered D. The device being powered D may be a transportable device used during MR imaging, and may be a module and/or telemetry device for measuring one or more parameter(s) such as, for example, oxygen saturation (SpO2), and electrocardiogram (ECG).

An induced current may be provided through the reconfigurable conductive coil 110 in FIG. 1A, and the induced current may generate magnetic flux given the inductive loop configuration of the reconfigurable conductive coil 110 in FIG. 1A.

FIG. 1B illustrates the reconfigurable charger for a device being powered of FIG. 1A in a second state, in accordance with a representative embodiment.

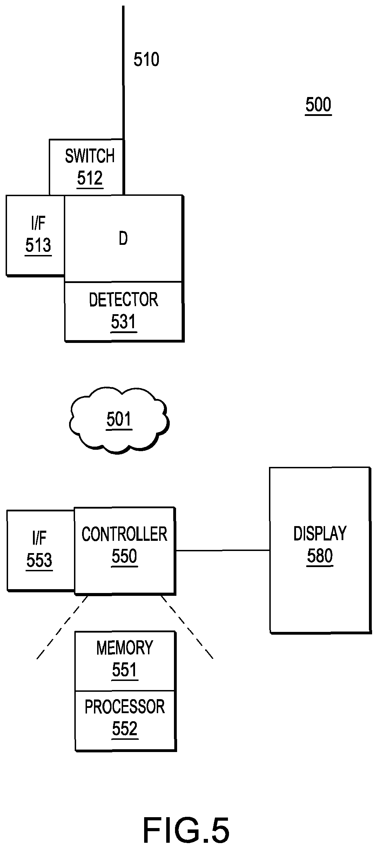

In FIG. 1B, the reconfigurable conductive coil 110 from FIG. 1A now has an open circuit that ensures no current is induced in the reconfigurable conductive coil 110. The reconfigurable charger that includes the reconfigurable conductive coil 110 may also include a switch as shown by the switch 512 in FIG. 5, so that the second physical configuration comprises an open circuit when the reconfigurable conductive coil 110 is opened via the switch. Accordingly, since a closed loop is required to be pierced by a magnetic flux in order to induce a current, and the loop is no longer closed, the magnetic flux induces no current flow.

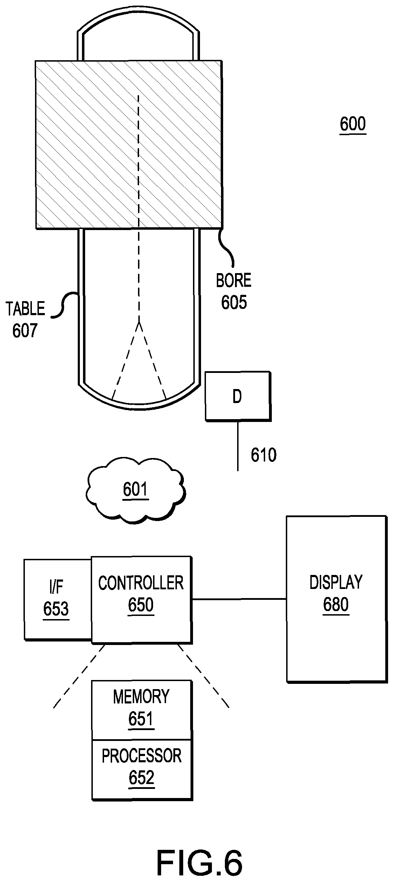

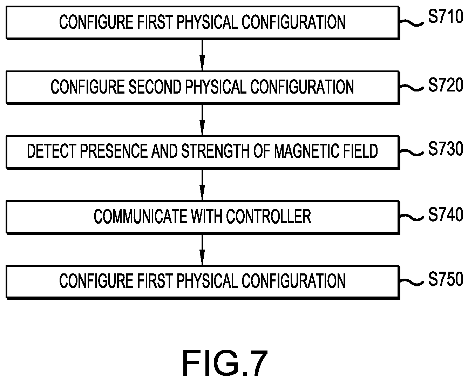

The reconfigurable conductive coil 110 may be part of the system 500 in FIG. 5 and/or the system 600 in FIG. 6, and configuration operations for the reconfigurable conductive coil 110 are shown in and described with respect to the method of FIG. 7.

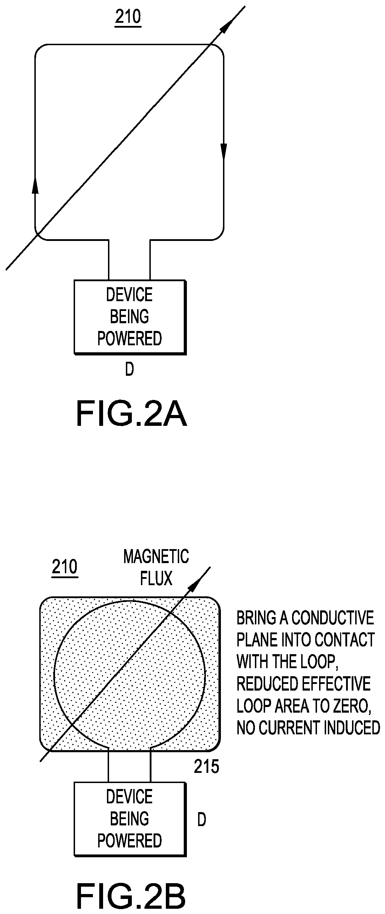

FIG. 2A illustrates a reconfigurable charger for a device being powered in a first state, in accordance with a representative embodiment.

FIG. 2A includes a reconfigurable conductive coil 210 attached to a device being powered D. The reconfigurable conductive coil 210 may be considered an element of the device being powered D, or may be considered to be separate and separable from the device being powered D. The device being powered D may be a transportable device used during MR imaging, and may be a module and/or telemetry device for measuring one or more parameter(s) such as, for example, oxygen saturation (SpO2), and electrocardiogram (ECG).

An induced current may be provided through the reconfigurable conductive coil 210 in FIG. 2A, and the induced current may generate magnetic flux given the loop configuration of the inductive loop in the reconfigurable conductive coil 210 in FIG. 2A.

FIG. 2B illustrates a reconfigurable charger for a device being powered of FIG. 2A in a second state, in accordance with a representative embodiment.

In FIG. 2B, the reconfigurable conductive coil 210 from FIG. 2A is brought into contact with a conductive plane 215 to ensure no current is induced in the reconfigurable conductive coil 210. The effective loop area of the inductive loop in the reconfigurable conductive coil 210 in FIG. 2B is reduced to zero by the conductive plane 215 so as to ensure that no current is induced in the reconfigurable conductive coil 210. Accordingly, the conductive plane completely electrically connects every point on the loop of wire to the same potential, thereby there is no loop area for the magnetic flux to pierce and no current is induced.

The reconfigurable conductive coil 210 may be part of the system 500 in FIG. 5 and/or the system 600 in FIG. 6, and configuration operations for the reconfigurable conductive coil 110 are shown in and described with respect to the method of FIG. 7.

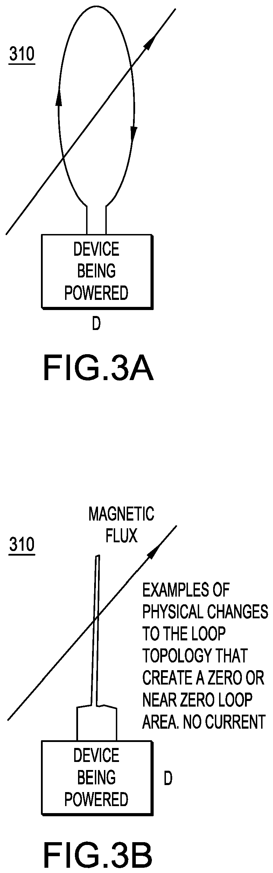

FIG. 3A illustrates a reconfigurable charger for a device being powered in a first state, in accordance with a representative embodiment.

FIG. 3A includes a reconfigurable conductive coil 310 attached to a device being powered D. The reconfigurable conductive coil 310 may be considered an element of the device being powered D, or may be considered to be separate and separable from the device being powered D. The device being powered D may be a transportable device used during MR imaging, and may be a module and/or telemetry device for measuring one or more parameter(s) such as, for example, oxygen saturation (SpO2), and electrocardiogram (ECG).

An induced current may be provided through the reconfigurable conductive coil 310 in FIG. 3A, and the induced current may generate magnetic flux given the loop configuration of the inductive loop in the reconfigurable conductive coil 310 in FIG. 3A.

FIG. 3B illustrates a reconfigurable charger for a device being powered of FIG. 3A in a second state, in accordance with a representative embodiment.

In FIG. 3B, the reconfigurable conductive coil 310 from FIG. 3A is flattened to eliminate the loop configuration of the inductive loop in the reconfigurable conductive coil 310 in FIG. 3A. The result of the reconfiguration in FIG. 3B is no cross-sectional area or substantially no cross-sectional area for the reconfigurable conductive coil 310. Substantially no cross-sectional area may be considered to refer to a relatively low cross-sectional area compared to the cross-sectional area in FIG. 3A, such as 5% or lower of the cross-sectional area in FIG. 3A. The reduction or elimination of the cross-sectional area by the reconfiguration in FIG. 3B ensures that no current or substantially no current is induced in the reconfigurable conductive coil 310. Accordingly, since the loop area is reduced to zero or very near zero, there is no magnetic flux piercing the loop and no current or a negligible current is induced.

The reconfigurable conductive coil 310 may be part of the system 500 in FIG. 5 and/or the system 600 in FIG. 6, and configuration operations for the reconfigurable conductive coil 110 are shown in and described with respect to the method of FIG. 7.

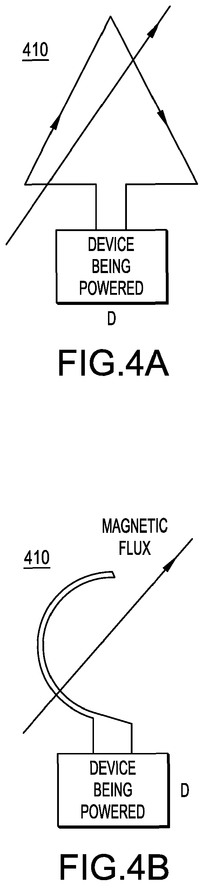

FIG. 4A illustrates a reconfigurable charger for a device being powered in a first state, in accordance with a representative embodiment.

FIG. 4A includes a reconfigurable conductive coil 410 attached to a device being powered D. The reconfigurable conductive coil 410 may be considered an element of the device being powered D, or may be considered to be separate and separable from the device being powered D. The device being powered D may be a transportable device used during MR imaging, and may be a module and/or telemetry device for measuring one or more parameter(s) such as, for example, oxygen saturation (SpO2), and electrocardiogram (ECG).

An induced current may be provided through the reconfigurable conductive coil 410 in FIG. 4A, and the induced current may generate magnetic flux given the loop configuration of the inductive loop in the reconfigurable conductive coil 410 in FIG. 4A.

FIG. 4B illustrates a reconfigurable charger for a device being powered of FIG. 4A in a second state, in accordance with a representative embodiment.

In FIG. 4B, the reconfigurable conductive coil 410 from FIG. 4A is folded to eliminate the loop configuration of the inductive loop in the reconfigurable conductive coil in FIG. 4A. The result of the reconfiguration in FIG. 4B is no cross-sectional area or substantially no cross-sectional area for the reconfigurable conductive coil 410. Substantially no cross-sectional area may again be considered to refer to a relatively low cross-sectional area compared to the cross-sectional area in FIG. 4A, such as 5% or lower of the cross-sectional area in FIG. 4A. The reduction or elimination of the cross-sectional area by the reconfiguration in FIG. 4B ensures that no current or substantially no current is induced in the reconfigurable conductive coil 410. Accordingly, since the loop area is reduced to zero or very near zero, there is no magnetic flux piercing the loop and no current or a negligible current is induced.

The reconfigurable conductive coil 410 may be part of the system 500 in FIG. 5 and/or the system 600 in FIG. 6, and configuration operations for the reconfigurable conductive coil 110 are shown in and described with respect to the method of FIG. 7.

FIG. 5 illustrates a system for a reconfigurable charger for a device being powered, in accordance with a representative embodiment.

The system 500 in FIG. 5 includes a reconfigurable charger as discussed above, having a reconfigurable conductive coil 510 attached to a device being powered D. The system 500 may also include a switch 512, an interface 513 and a magnetic detector 531 all physically connected directly or indirectly to the device being powered D. The reconfigurable conductive coil 510 may be any of the reconfigurable conductive coils shown in FIG. 1A and FIG. 1B, in FIG. 2A and FIG. 2B, in FIG. 3A and FIG. 3B, or in FIG. 4A and FIG. 4B. Of course, the reconfigurable conductive coils described herein are not particularly limited to the embodiments shown herein, and other forms of reconfigurable conductive coils may be used so long as consistent with the teachings herein.

The system 500 may also include a network 501, a controller 550, and/or a display 580. The controller 550 may include a memory 551, a processor 552, and an interface 553.

The switch 512 is an element of the reconfigurable charger that includes the reconfigurable conductive coil 510. The second physical configuration for the reconfigurable conductive coil 510 may comprise an open circuit when the reconfigurable conductive coil 510 is opened via the switch 512.

The interface 513 and the interface 553 may each be wireless interfaces. The controller 550 may interact wirelessly with the switch 512 and the magnetic detector 531 via the interface 513 and the interface 553. The network 501 may comprise a wireless local area network (LAN) such as a wireless fidelity (WiFi) network. The interface 513 and/or the interface 553 may include wireless antennas, or other types of receiver circuitry that connect the controller 550 to the interface 513 and other electronic elements. The interface 553 may also include user interfaces such as buttons, keys, a mouse, a microphone, a speaker, a display separate from the display 580, or other elements that users can use to interact with the controller 550 such as to enter instructions and receive output.

The memory 551 stores instructions and the processor 552 executes the instructions. A controller 150 may include more elements than depicted in FIG. 5. The memory 551 may include a set of software instructions that can be executed to cause the controller 550 to perform aspects of methods or computer-based functions disclosed herein. The controller 150 may operate as a standalone device or may be connected, for example, using the network 501, to other computer systems or peripheral devices. In embodiments, the controller 550 performs logical processing based on digital signals received via an analog-to-digital converter. The controller 550 can also be implemented as or incorporated into various devices, such as a workstation that includes a controller, a stationary computer, a mobile computer, a personal computer (PC), a laptop computer, a tablet computer, or any other machine capable of executing a set of software instructions (sequential or otherwise) that specify actions to be taken by that machine. The controller 550 can be incorporated as or in a device that in turn is in an integrated system that includes additional devices. Further, while the controller 550 is illustrated in the singular, the controller 550 may be implemented in a "system" that includes any collection of systems or sub-systems that individually or jointly execute a set, or multiple sets, of software instructions to perform one or more computer functions.

The processor 552 may be considered a representative example of a processor of a controller and executes instructions to implement some or all aspects of methods and processes described herein. The processor 552 is tangible and non-transitory. As used herein, the term "non-transitory" is to be interpreted not as an eternal characteristic of a state, but as a characteristic of a state that will last for a period. The term "non-transitory" specifically disavows fleeting characteristics such as characteristics of a carrier wave or signal or other forms that exist only transitorily in any place at any time. The processor 552 is an article of manufacture and/or a machine component. The processor 552 is configured to execute software instructions to perform functions as described in the various embodiments herein. The processor 552 may be a general-purpose processor or may be part of an application specific integrated circuit (ASIC). The processor 552 may also be a microprocessor, a microcomputer, a processor chip, a controller, a microcontroller, a digital signal processor (DSP), a state machine, or a programmable logic device. The processor 552 may also be a logical circuit, including a programmable gate array (PGA), such as a field programmable gate array (FPGA), or another type of circuit that includes discrete gate and/or transistor logic. The processor 552 may be a central processing unit (CPU), a graphics processing unit (GPU), or both. Additionally, any processor described herein may include multiple processors, parallel processors, or both. Multiple processors may be included in, or coupled to, a single device or multiple devices.

The term "processor" as used herein encompasses an electronic component able to execute a program or machine executable instruction. References to a system or a controller comprising "a processor" should be interpreted to include more than one processor or processing core, as in a multicore processor or multiple separate processors. A processor may also refer to a collection of processors within a single computer system or distributed among multiple computer systems. Programs have software instructions performed by one or multiple processors that may be within the same computing device or which may be distributed across multiple computing devices.

The memory 551 may be representative of memories in the system 500, such as a main memory and a static memory, where memories in the controller 550 and the system 500 communicate with each other and the processor 552 via a bus. The memory 551 stores instructions used to implement some or all aspects of methods and processes described herein. Memories described herein are tangible storage mediums for storing data and executable software instructions and are non-transitory during the time software instructions are stored therein. As used herein, the term "non-transitory" is to be interpreted not as an eternal characteristic of a state, but as a characteristic of a state that will last for a period. The term "non-transitory" specifically disavows fleeting characteristics such as characteristics of a carrier wave or signal or other forms that exist only transitorily in any place at any time. The memory 551 is an article of manufacture and/or machine components. The memory 551 is a computer-readable medium from which data and executable software instructions can be read by a computer (e.g., the processor 552). The memory 551 may be implemented as one or more of random access memory (RAM), read only memory (ROM), flash memory, electrically programmable read only memory (EPROM), electrically erasable programmable read-only memory (EEPROM), registers, a hard disk, a removable disk, tape, compact disk read only memory (CD-ROM), digital versatile disk (DVD), floppy disk, blu-ray disk, or any other form of storage medium known in the art. The memories may be volatile or non-volatile, secure and/or encrypted, unsecure and/or unencrypted.

"Memory" is an example of a computer-readable storage medium. Computer memory is any memory which is directly accessible to a processor. Examples of computer memory include, but are not limited to RAM memory, registers, and register files. References to "computer memory" or "memory" should be interpreted as possibly being multiple memories. The memory may for instance be multiple memories within the same computer system. The memory may also be multiple memories distributed amongst multiple computer systems or computing devices.

In an embodiment, dedicated hardware implementations, such as application-specific integrated circuits (ASICs), field programmable gate arrays (FPGAs), programmable logic arrays and other hardware components, are constructed to implement one or more of the methods described herein. One or more embodiments described herein may implement functions using two or more specific interconnected hardware modules or devices with related control and data signals that can be communicated between and through the modules. Accordingly, the present disclosure encompasses software, firmware, and hardware implementations. Nothing in the present application should be interpreted as being implemented or implementable solely with software and not hardware such as a tangible non-transitory processor and/or memory.

The display 580 may be local to the controller 550 or may be remotely connected to the controller 550. The display 580 may be connected to the controller 550 via a local wired interface such as an Ethernet cable or via a local wireless interface such as a Wi-Fi connection. The display 580 may be interfaced with other user input devices by which users can input instructions, including mouses, keyboards, thumbwheels and so on. The display 580 may be a monitor such as a computer monitor, a display on a mobile device, an augmented reality display, a television, an electronic whiteboard, or another screen configured to display electronic imagery. The display 580 may also include one or more input interface(s) that may connect to other elements or components, as well as an interactive touch screen configured to display prompts to users and collect touch input from users.

The controller 550 may perform some aspects of operations described herein directly or indirectly. For example, the controller 550 may indirectly control operations such as by generating and transmitting content to be displayed on the display 580. The controller 550 may directly control other operations such as logical operations performed by the processor 552 executing instructions from the memory 551 based on input received from electronic elements and/or users via the interfaces. Accordingly, the processes implemented by the controller 550 when the processor 552 executes instructions from the memory 551 may include steps not directly performed by the controller 550.

FIG. 6 illustrates a system for a reconfigurable charger for a device being powered, in accordance with a representative embodiment.

FIG. 6 is a duplicate of FIG. 5 except that FIG. 6 also shows the context of a magnetic resonance imaging bore 605 and a table 607, and some of the elements of FIG. 5 are omitted for the sake of brevity. The system 600 in FIG. 6 includes a reconfigurable conductive coil 610 attached to a device being powered D. The system 600 also includes a network 601, a controller 650, and a display 680. The controller 650 includes a memory 651 and a processor 652, and an interface 653. The reconfigurable conductive coil 610 may be any of the reconfigurable conductive coils shown in FIG. 1A and FIG. 1B, in FIG. 2A and FIG. 2B, in FIG. 3A and FIG. 3B, or in FIG. 4A and FIG. 4B.

The magnetic resonance imaging bore 605 is an aspect of a cylindrical magnet used in the system 600 as a magnetic resonance imaging system. The cylindrical magnet with the magnetic resonance imaging bore 605 may be the outermost functional element around the magnetic resonance imaging bore 605 in the system 600. Other elements of the magnetic resonance imaging system may be arranged in the magnetic resonance imaging bore 605 directly or indirectly on the table 607. The patient being imaged is placed on the table 607 and the table 607 is manipulated in the magnetic resonance imaging bore 605. The cylindrical magnet which includes the magnetic resonance imaging bore 605 is only an example, as other types of magnets can be used for magnetic resonance imaging systems, including a split cylindrical magnet or an open magnet. Other elements arranged in the magnetic resonance imaging bore 605 may include a cylindrical body coil immediately interior to the cylindrical magnet, wherein the body coil provides the main uniform, static, magnetic field that excites and aligns the hydrogen atoms. Examples of uniform magnetic fields provided by a body coil are 1.5 Teslas, 3.0 Teslas, or 7.0 Teslas. The system 600 may also include field gradient coils immediately interior to the body coil, wherein the field gradient coils are relatively low frequency coils used to choose a plane of interest relative to X, Y and Z axes. Such field gradient coils are used to generate a magnetic field across the subject in the magnetic resonance imaging bore 605 by using a field gradient. The system 600 may also include a radio frequency coil immediately interior to the field gradient coil. The radio frequency coil is used to deliver the B1 field to selected slices of an imaging zone, to result in the delivery of the B 1 field to manipulate orientations of magnetic spins of hydrogen nuclei within the imaging zone. The radio frequency coil is used in the transmit stage, and may be used in some systems for the receive stage. Once a transmit cycle is complete, the hydrogen atoms return to original positions, and emanate a weak radio frequency signal. This weak radio frequency signal from the hydrogen atoms returned to their original positions is what is picked up in the receive stage of the magnetic resonance imaging cycle.

The table 607 passes through the magnetic resonance imaging bore 605 of the system 600. A guidance system may be provided to guide the table 607, and may be a track, a rut or a rail fixture.

The reconfigurable conductive coil 610 is representative of at least one reconfigurable conductive coil, and is configured to have a first physical configuration and a second physical configuration. The second physical configuration is for when the reconfigurable conductive coil is in the magnetic resonance imaging bore 605 of the magnetic resonance imaging system. The first physical configuration is for recharging the reconfigurable conductive coil 610 when the reconfigurable conductive coil 610 is distal from the magnetic resonance imaging system. The reconfigurable charger that includes the reconfigurable conductive coil 610 is configured to wirelessly recharge a peripheral device D in the first physical configuration. The second physical configuration provides a reduction in induced current in the reconfigurable conductive coil 610 compared to the first physical configuration.

FIG. 7 illustrates a method of operation for a reconfigurable charger for a device being powered, in accordance with a representative embodiment.

The method of FIG. 7 may be performed by the system 500 including the controller 550 and any of the reconfigurable chargers from earlier embodiments including FIG. 1A and FIG. 1B, or FIG. 2A and FIG. 2B, or FIG. 3A and FIG. 3B, or FIG. 4A and FIG. 4B.

At S710, a first physical configuration is configured for the reconfigurable charger. The first physical configuration may be the configuration shown in FIG. 1A or FIG. 2A or FIG. 3A or FIG. 4A, and should result in an ability for current to run through the reconfigurable conductive coil of the reconfigurable charger. The first physical configuration may be for recharging when the reconfigurable charger is distal from the magnetic resonance imaging system.

At S720, a second physical configuration is configured for a reconfigurable charger. The second physical configuration may be the configuration shown in FIG. 1B or FIG. 2B, or FIG. 3B or FIG. 4B, and should result in no current or insignificant current in the reconfigurable charger, such as when in the magnetic resonance imaging bore 605 or when the patient is moving towards the magnetic resonance imaging bore 605.

At S730, presence and strength of a magnetic field is detected. For example, the magnetic detector 531 may detect presence and strength of a magnetic field. For example, the magnetic detector 531 may automatically detect the presence ad strength of the magnetic field, so as to communicate with the controller 550 or the controller 650 based on detecting the presence and strength of the magnetic field to change between the configurations, such as between the second physical configuration and the first physical configuration. The controller 550 or the controller 650 may then instruct the switch 512 to open the circuit of the reconfigurable coil such that the second physical configuration will comprise an open circuit when the reconfigurable conductive coil 510 or the reconfigurable conductive coil 610 is opened via the switch 512.

At S740, the method of FIG. 7 includes communicating with a controller such as the controller 550 in FIG. 5. For example, the interface 513 may initiate a communication with the controller 550 to indicate the detected presence and strength of the magnetic field.

At S750, the first physical configuration may again be configured for the reconfigurable charger.

In accordance with various embodiments of the present disclosure, the methods described herein may be implemented using a hardware computer system that executes software programs. Further, in an exemplary, non-limited embodiment, implementations can include distributed processing, component/object distributed processing, and parallel processing. Virtual computer system processing may implement one or more of the methods or functionalities as described herein, and a processor described herein may be used to support a virtual processing environment.

Accordingly, magnetic resonance compatible wireless charging enables wireless recharging for transportable devices used in the MR environment using inductive coupling. Reconfigurable conductive coils may be used to enable the wireless recharging. The wireless recharging described herein may prevent the damage that would result from an inductive loop in a conductive coil in the MR environment by changing the properties of the inductive loop in the conductive coil such that it can operate like a standard loop when recharging away from the MR environment yet generate zero or near zero current, voltage and heat when configured for operations in the MR environment.

Although magnetic resonance compatible wireless charging has been described with reference to particular means, materials and embodiments, magnetic resonance compatible wireless charging is not intended to be limited to the particulars disclosed; rather magnetic resonance compatible wireless charging extends to all functionally equivalent structures, methods, and uses such as are within the scope of the appended claims.

The illustrations of the embodiments described herein are intended to provide a general understanding of the structure of the various embodiments. The illustrations are not intended to serve as a complete description of all of the elements and features of the disclosure described herein. Many other embodiments may be apparent to those of skill in the art upon reviewing the disclosure. Other embodiments may be utilized and derived from the disclosure, such that structural and logical substitutions and changes may be made without departing from the scope of the disclosure. Additionally, the illustrations are merely representational and may not be drawn to scale. Certain proportions within the illustrations may be exaggerated, while other proportions may be minimized. Accordingly, the disclosure and the figures are to be regarded as illustrative rather than restrictive.

One or more embodiments of the disclosure may be referred to herein, individually and/or collectively, by the term "invention" merely for convenience and without intending to voluntarily limit the scope of this application to any particular invention or inventive concept. Moreover, although specific embodiments have been illustrated and described herein, it should be appreciated that any subsequent arrangement designed to achieve the same or similar purpose may be substituted for the specific embodiments shown. This disclosure is intended to cover any and all subsequent adaptations or variations of various embodiments. Combinations of the above embodiments, and other embodiments not specifically described herein, will be apparent to those of skill in the art upon reviewing the description.

In the claims, any reference signs placed between parentheses shall not be construed as limiting the claim.

## Claims

1. A reconfigurable wireless charger component comprising:
at least one reconfigurable conductive coil (110, 210, 310, 410) configured to have a first physical configuration for recharging when distal from a magnetic system, and to have a second physical configuration when close to a magnetic system, wherein,
the reconfigurable wireless charger component in the first physical configuration is configured to wirelessly recharge a device (D), and
the second physical configuration provides a reduction in induced current in the at least one reconfigurable conductive coil (110) compared to the first physical configuration.

2. The reconfigurable wireless charger component of claim 1, wherein the reduction in induced current is obtained by reducing a loop area of the reconfigurable conductive coil, wherein the reduction of the loop area is enabled by opening the inductive loop or changing topology of the inductive loop.

3. The reconfigurable wireless charger component of claim 1 or 2, further comprising:
a switch (512), wherein the second physical configuration comprises an open circuit when the at least one reconfigurable conductive coil (110) is opened via the switch (512).

4. The reconfigurable wireless charger component of any of the preceding claims, further comprising:
a conductive plane (215) configured to be brought into contact with the at least one reconfigurable conductive coil (210) in the second physical configuration.

5. The reconfigurable wireless charger component of any of the preceding claims, wherein the first physical configuration provides a first cross-sectional area for the at least one reconfigurable conductive coil, and the second physical configuration provides a second cross-sectional area for the at least one reconfigurable conductive coil, the second cross-sectional area being significantly smaller than the first cross-sectional area.

6. The reconfigurable wireless charger component of claim 5, wherein the at least one reconfigurable coil is configured to fold or flatten such that the second cross-sectional area is substantially zero.

7. A system (500) for reconfigurable charging in a magnetic resonance environment, the system comprising:
a reconfigurable wireless charger component according to claims 1-6, and
a controller (550) configured to control the at least one reconfigurable conductive coil (110, 210, 310, 410) of the reconfigurable charger to change between the first physical configuration and the second physical configuration.

8. The system (500) of claim 7, further comprising:
a magnetic detector (531) that detects presence and/or strength of a magnetic field and communicates with the controller (550) to make the controller control the at least one reconfigurable conductive coil to change between the first physical configuration and the second physical configuration, based on the presence and/or strength of the magnetic field.

9. A method of operating a reconfigurable wireless charger component in a magnetic environment, the method comprising:
configuring at least one reconfigurable conductive coil (110, 210, 310, 410) to have a first physical configuration for recharging when distal from a magnetic system (S710);
configuring the at least one reconfigurable conductive coil to have a second physical configuration when close to the magnetic system (S720);
wirelessly recharging a device (D) via the at least one reconfigurable conductive coil in the first physical configuration,
wherein the second physical configuration provides a reduction in induced current in the at least one reconfigurable conductive coil (110) compared to the first physical configuration.

10. The method of claim 9, further comprising:
controlling, via a controller (550), the at least one reconfigurable conductive coil to change between the first physical configuration and the second physical configuration.

11. The method of claim 10, further comprising:
detecting, via a magnetic detector (531), presence and/or strength of a magnetic field (S730); and
communicating, between the magnetic detector (531) and the controller (S740) to make the controller control the at least one reconfigurable conductive coil to change between the first physical configuration and the second physical configuration, based on the presence and/or strength of the magnetic field.

12. The method of claim 10 or 11, further comprising:
controlling, by the controller, a switch (512), wherein the second physical configuration comprises an open circuit when the at least one reconfigurable conductive coil (110) is opened via the switch.

13. The method of any of claims 10-12, further comprising:
controlling, by the controller, a conductive plane (215) to be brought into contact with the at least one reconfigurable conductive coil in the second physical configuration.

14. The method of any of claims 10-13, further comprising:
controlling by the controller the at least one reconfigurable coil to fold or flatten, such that the first physical configuration provides a firts cross-sectional area for the at least one reconfigurable conductive coil (310, 410), and the second physical configuration provides a second cross-sectional area for the at least one reconfigurable conductive coil, the second cross-sectional area being significantly smaller than the first cross-sectional area.

15. A computer program product comprising instructions which, when the program is executed by a system according to any of claims 7-8, cause the system to carry out the method of any of claims 10-14.
